Europäisches Patentamt

(19) European Patent Office

Office européen des brevets

(11) **EP 0 603 409 B1**

(12) **EUROPEAN PATENT SPECIFICATION**

(45) Date of publication and mention
of the grant of the patent:
**13.08.1997 Bulletin 1997/33**

(21) Application number: **93913535.6**

(22) Date of filing: **17.06.1993**

(51) Int. Cl.$^6$: **B01J 31/02**, C07D 309/30,
C07B 61/00

(86) International application number:
**PCT/JP93/00815**

(87) International publication number:
**WO 93/25308 (23.12.1993 Gazette 1993/30)**

(54) **HYDRATED ZIRCONIUM OXIDE MODIFIED WITH ORGANO-SILICON COMPOUND AND CATALYSTS FOR ALCOHOL OXIDATION AND LACTONE PRODUCTION**

MIT EINER ORGANOSILICIUMVERBINDUNG MODIFIZIERTES, HYDRATIERTES ZIRKONIUMOXYD UND KATALOGYSATOREN FÜR ALKOHOLOXYDATION UND FÜR LACTONHERSTELLUNG

OXYDE DE ZIRCONIUM HYDRATE MODIFIE AVEC UN COMPOSE ORGANOSILICIEN ET CATALYSEURS UTILISES DANS L'OXYDATION D'UN ALCOOL ET LA PRODUCTION DE LACTONE

(84) Designated Contracting States:
**DE FR GB**

(30) Priority: **17.06.1992 JP 181564/92**
**18.06.1992 JP 182907/92**

(43) Date of publication of application:
**29.06.1994 Bulletin 1994/26**

(73) Proprietor: **JAPAN TOBACCO INC.**
**Shinagawa-ku, Tokyo 140 (JP)**

(72) Inventors:
• **KUNO, Hideyuki,**
**Japan Tobacco Inc.**
**Midori-ku, Yokohama-shi Kanagawa-ken 227**
**(JP)**
• **SHIBAGAKI, Makoto,**
**Japan Tobacco Inc.**
**Midori-ku, Yokohama-shi Kanagawa-ken 227**
**(JP)**
• **TAKAHASHI, Kyoko,**
**Japan Tobacco Inc.**
**Midori-ku, Yokohma-shi Kanagawa-ken 227 (JP)**
• **MATSUSHITA, Hajime,**
**Japan Tobacco Inc.**
**Midori-ku, Yokohama-shi Kanagawa-ken 227**
**(JP)**

(74) Representative: **Ruffles, Graham Keith**
**MARKS & CLERK,**
**57-60 Lincoln's Inn Fields**
**London WC2A 3LS (GB)**

(56) References cited:
**EP-A- 0 028 107          DE-A- 2 313 073**
**JP-A-61 204 143**

• **DATABASE WPI Week 8141, Derwent**
**Publications Ltd., London, GB; AN 81074679 &**
**JP-A-56 108 524 (HITACHI KK) 28 August 1981**

EP 0 603 409 B1

**Description**

FIELD OF THE INVENTION

The present invention relates to novel hydrous zirconium oxide and utilities thereof. The present invention relates more particularly to hydrous zirconium oxide modified with an organosilicon compound and the use of said hydrous zirconium oxide as a catalyst for oxidation of alcohols and as a catalyst for production of lactones, particularly lactones having a middle or large ring.

BACKGROUND OF THE INVENTION

The hydrous zirconium oxide acts as a catalyst for a wide variety of reactions. The main reactions catalyzed by the hydrous zirconium oxide are e.g. esterification and alcohol oxidation. Esterification proceeds intermolecularly in high yield. However, the problem of hydrous zirconium oxide is low selectivity as a catalyst for intramolecular esterification. In addition, there are problems such as frequent occurrence of side reactions and low selectivity as a catalyst for oxidation of alcohols, particularly oxidation of primary alcohols.

Hence, an object of the present invention is to provide novel hydrous zirconium oxide modified with an organosilicon compound, which is useful as a catalyst for alcohol oxidation and lactone production.

Hereinafter, the technical background of the present invention is described.

(1) Technical background of catalysts for oxidation of alcohols

Generally, an alcohol is oxidized to produce the corresponding aldehyde or ketone. For such alcohol oxidation, a secondary alcohol can be relatively easily oxidized to the corresponding ketone according to "Oppenauer oxidation", i.e. a method for oxidation of a secondary alcohol into the corresponding ketone in the presence of metallic alkoxide such as aluminum isopropoxide, etc., and a ketone as an oxidizing agent

As for a primary alcohol, however, the production of the corresponding aldehyde by oxidation is significantly difficult.

That is, Collins oxidation (TETRAHEDRON LETTERS, p. 3363, 1968) and the DMSO-DCC method (JOURNAL OF THE AMERICAN CHEMICAL SOCIETY, vol. 85, 1963) are conventionally known as methods of oxidation of the primary alcohol, in which an oxidized product from the primary alcohol requires troublesome procedures of purification. Furthermore, environmental pollution must particularly be taken into consideration because of the difficult recovery and re-use of the reaction reagents.

Another method, which similar to Oppenauer oxidation comprises use of aluminum isopropoxide as a catalyst and quinone as an oxidizing agent, is known for converting the primary alcohol into the corresponding aldehyde (ORGANIC REACTIONS, vol. 6, p. 207, 1951). Even in this method, there are problems such as difficult re-use of catalyst, troublesome purification procedures after reaction, etc.

There is another proposed method in which an alcohol is allowed to react with solid bromous acid salt in the presence of alumina or silica as a heterogeneous catalyst to produce the corresponding aldehyde (Japanese Published Unexamined Patent Application No. 151,532/89). However, it cannot be denied that the practical value of this method is relatively low due to its applicability limited to benzyl alcohols.

Hence, another object of the present invention is to provide a catalyst for use in the oxidation of alcohols for production of aldehydes or ketones, in which the procedures of alcohol oxidation and product purification are very easy, the catalyst can be re-used, and no environmental pollution is caused.

(2) Background of catalysts for production of lactones

Like ε-caprolactone with a 7-membered ring and 15-pentadecanolide with a 16-membered ring, not a few compounds with a lactone ring have been used as perfumes from the old days.

Further, picromycin with a 14-membered ring and its glycoside, erythromycin, are known as antibiotics. In addition, Zearalenon, Lasiodiplodin, Curvularin, brefeldin A, for example, are known as antibiotics having a large lactone ring.

Conventionally, the use of lipase (hydrolase) as a catalyst is known as a typical means of efficiently producing large ring lactones (TETRAHEDRON LETTERS, vol. 28, p. 805, 1987).

In such a method, however, hydroxy acids as starting material should be diluted to high degree for prevention of intermolecular reaction in production of middle- or large-ring lactones, which leads to troublesome procedures in large-scale synthesis of the lactones. Moreover, reaction selectivity is not so high and durability of enzyme activity not so long, resulting in very poor yield of middle-ring lactones (8-11-membered rings) and large-ring lactones (not less than 12-membered rings).

Therefore, a further object of the present invention is to provide a catalyst for use in a process in which middle- and large-ring lactones can be produced efficiently and easily.

SUMMARY OF THE INVENTION

As a result of eager research in view of the above technical background, the present inventors found novel hydrous zirconium oxide modified with an organosilicon compound, and also that said hydrous zirconium oxide can be used as an catalyst significantly excellent in oxidation of alcohols or production of lactones.

That is, the present invention comprises:

(1) Hydrous zirconium oxide modified with an organosilicon compound, wherein 50-90 % of the hydroxyl groups on the surface of the hydrous zirconium oxide are substituted by the organosilicon compound.

(2) A catalyst for the oxidation of alcohols, comprising as a constituent the hydrous zirconium oxide modified with an organosilicon compound as defined in (1).

(3) A catalyst for the production of lactones, comprising as a constituent the hydrous zirconium oxide modified with an organosilicon compound as defined in (1).

DETAILED DESCRIPTION OF THE INVENTION

A. Production of the hydrous zirconium oxide of the present invention

In the present invention, "the hydrous zirconium oxide modified with an organosilicon compound" (hereinafter referred to as "the hydrous zirconium oxide of the invention") is obtained in a process in which hydrous zirconium oxide produced by the method described by Mizusaki et al. in JP-A-63 226 141 is allowed to react (e.g. through coupling reaction) with an organosilicon compound. The hydrous zirconium oxide referred to herein is a solid catalyst with hydroxyl groups on the surface. These hydroxyl groups are considered to act as activation sites responsible for the occurrence of various side reactions. For inhibition of side effects, such surface hydroxyl groups are allowed to react so as to be covered with (i.e. substituted by) an organosilicon compound, whereby a catalyst of higher selectivity can be obtained.

Examples of the organosilicon compound are trimethylsilyl chloride, dimethyloctadecylsilyl chloride, triethylsilyl chloride, 3-aminopropyltriethoxysilane and diphenyldiethoxysilane, among which trimethylsilyl chloride is preferable for relatively low steric hindrance of its molecule to facilitate higher modification proportion of surface hydroxyl groups.

For coverage of the hydrous zirconium oxide, the organosilicon compound can be used as such in coupling reaction with a hydroxyl group on the hydrous zirconium oxide. Alternatively, the organosilicon compound is added to an inert organic solvent such as diethyl ether, hexane, benzene, toluene and xylene, and the hydrous zirconium oxide is immersed therein and then dried, whereby the hydrous zirconium oxide modified with the desired organosilicon compound can be obtained.

The coupling reaction in preparing the hydrous zirconium oxide of the invention can be suitably effected in the liquid phase batch method, stationary bed flow method, or vacuum deposition method.

For the liquid batch method, the temperature for coupling reaction is selected in the range of 10-300 °C and it is conveniently in the range of room temperature to 100 °C i.e. under the boiling points of many solvents commercially available as liquid phase.

For the stationary bed flow method, the coupling reaction can be carried out at a predetermined temperature in the range of 10-450 °C. In consideration of compound stability and reaction efficiency, the coupling reaction is preferably carried out in the range of around room temperature to 300 °C, preferably room temperature or thereabout for assurance of compound stability in cases where chloride-type compounds are used as organosilicon compounds.

To keep high reactivity with surface hydrogen groups, the reaction temperature for alkoxysilane-type compounds is preferably in the range of 100-300 °C.

The catalyst obtained in the above reaction is a white solid. XPS analysis (as described below) and analysis of amounts of surface hydroxyl groups on the present catalyst indicates that the organosilicon compound is covalently bound to a hydroxyl group on the hydrous zirconium oxide.

50-90 % of the hydroxyl groups on the surface of the hydrous zirconium oxide of the invention are covered with the organosilicon compound. 70-85 % surface modification is preferable. Generally, hydroxyl groups on the surface act as activation sites for dehydration and aldol reaction. Therefore, 50 % or less of surface modification is not preferable owing to higher occurrence of side reactions and lower reaction yield. On the other hand, 90 % or more surface modification is difficult to attain.

The degree of surface modification can be measured according to a conventional method. An embodiment of such a measurement is specifically described in the following examples.

B. Alcohol oxidation using the hydrous zirconium oxide of the invention as a catalyst

The oxidation of alcohols can be effected in the presence of the hydrous zirconium oxide of the invention as a catalyst prepared in the manner as described above.

Alcohol oxidation is shown in reaction (1):

$$RR'CHOH \rightarrow RR'CO \tag{1}$$

wherein R and R' include, and are not particularly limited to, a hydrogen atom, substituted or unsubstituted alkyl, substituted or unsubstituted cycloalkyl, and substituted or unsubstituted aromatic groups.

Examples of the substituted or unsubstituted alkyl group are methyl, ethyl, n-propyl, i-propyl, and monochloromethyl groups, preferably methyl and ethyl groups.

Examples of the substituted or unsubstituted cycloalkyl group are cyclohexyl, cycloheptyl, cyclopentyl, 2-chlorocyclohexyl and 2-methylcyclohexyl groups, preferably cyclopentyl and cyclohexyl groups.

Examples of the substituted or unsubstituted aromatic group are are phenyl, p-toluyl and p-chlorophenyl groups, preferably phenyl and p-chlorophenyl groups.

The oxidizing agent used in the above reaction may be any kinds of aldehyde and ketone. For example, the aldehyde may be non-aromatic aldehydes such as pivalaldehyde or hexyl aldehyde, and aromatic aldehydes such as m-tolualdehyde, p-chlorobenzaldehyde or benzaldehyde, among which m-tolualdehyde and benzaldehyde are preferable for minimizing side reactions.

The ketone may be noncyclic ketones, such as acetone, diethyl ketone or diisopropyl ketone, ketones with a carbon ring or aromatic ring, such as benzophenone or cyclohexanone, among which benzophenone is preferable for minimizing side reactions.

The alcohol oxidation may be carried out in liquid phase as well as vapor phase.

Where liquid phase reaction is used for oxidation, the content of oxidizing agent in a starting material solution containing a solvent is 0.1-100 (W/V) %, and it is preferably 1.0-50 (W/V) for minimizing side reactions. The molar ratio of alcohol (as starting material)/oxidizing agent can be suitably selected in the range of 1/1 to 1/1000, and it is preferably from 1/50 to 1/1000 for attaining as high yield as possible.

For oxidation, a diluent not directly participating in the oxidation can be added. The type of diluent is not particularly limited as far as it does not affect the reaction system for oxidation.

In the absence of the diluent, the aldehyde or ketone formed further undergoes aldol condensation, resulting in lower yield of the desired aldehyde or ketone. Therefore, use of the diluent is essential for the reaction system.

The diluent used is an inert organic solvent, such as benzene, xylene, toluene, tetrahydrofuran, dioxane, diethylene glycol and diethyl ether and a mixture thereof.

The temperature for oxidation can be suitably selected in the range of 50-200 °C and it is desirably 80-150 °C for facilitating the procedure.

After oxidation, the solids in the reaction mixture are filtered off, and the filtrate is separated by fractional distillation into the desired aldehyde or ketone of high purity.

Where the flow method is used for oxidation, oxidation can be suitably carried out in the following manner.

For oxidation in the flow method, a reaction tube is charged with the catalyst of the invention prepared above in A. A solution containing the alcohol (i.e., RR'CHOH as described above) as a starting material, the above-mentioned oxidizing agent, and as necessary, the above-enumerated inert organic solvent is prepared, which is then vaporized for continuous introduction with a suitable carrier gas into the reaction tube having been heated to a predetermined temperature. The starting material continuously fed into above reaction tube comes in contact with the catalyst of the present invention, thereby yielding a reaction product which is then cooled on ice in the outlet of the reaction tube so that the effluent product is condensed for recovery.

In the flow method, the temperature for oxidation is in the range of 20-300 °C, preferably 100-200 °C.

If the flow method is used for oxidation, the content of oxidizing agent in a starting material solution containing a solvent is 0.1-100 (W/V) %, and it is preferably 1.0-50 (W/V) % for minimizing side reactions. The molar ratio of alcohol/oxidizing agent can be suitably selected in the range of 1/1-1/1000, and it is preferably 1/50-1/1000 for attaining as high yield as possible.

In the manner as described above, the hydrous zirconium oxide of the invention is used as a catalyst for efficient oxidation of an alcohol into the corresponding aldehyde or ketone.

In addition, the present catalyst can be reused in the production of alcohol.

C. Process for production of lactones using the hydrous zirconium oxide of the invention as a catalyst

Lactones can be effectively produced by the use of the hydrous zirconium oxide of the invention as a catalyst prepared in the above method.

As starting materials for production of lactones, hydroxy acids and derivatives thereof can be represented by formula (2):

$$OH\text{-}A\text{-}COOR'' \tag{2}$$

In formula (2), R'' is hydrogen or an alkyl group, and A is a substituted or unsubstituted alkylene group with 3 or more carbon atoms which is a straight chain or branched chain.

The alkyl group represented by R'' may be a methyl, ethyl, n-propyl, i-propyl, or n-butyl group, among which a methyl or ethyl group is preferable.

The number of carbon atoms in the main chain of the alkylene group A is preferably 2-20.

If the alkylene group is substituted with a substituent group, then the substituent group may be any group not reacting with carboxylate and hydroxyl groups, and specific examples are alkyl, phenyl, etc.

In the production of lactones using the hydrous zirconium oxide of the invention as a catalyst, hydroxy acids of formula (2) or derivatives thereof are diluted with an inert solvent including, but not limited to, toluene, xylene, tetrahydrofuran, diethyl ether, for example, among which toluene and xylene are preferable for attaining maximal yield.

The above-enumerated inert solvents can be used singly or in combination.

The concentration of hydroxyl acids or derivatives thereof in the organic solvent is usually 1 mM to 1 M, and it is preferably 6 mM to 300 mM for attaining higher reaction efficiency and higher yield of lactone.

Hydroxy acids or derivatives thereof are diluted with the inert solvent in the above concentration range, and their intramolecular esterification is effected in the presence of the hydrous zirconium oxide of the invention.

Although an embodiment of intramolecular esterification is not particularly limited, intramolecular esterification should be carried out in the flow reaction, but not in the liquid phase batch reaction.

For intramolecular esterification, the above starting material solution is supplied to the flow reaction system including the hydrous zirconium oxide of the invention immobilized as a catalyst for esterification, and after past through the reaction apparatus, the product is trapped by cooling.

The esterification is carried out usually at 25-400 °C, preferably 200-300 °C.

After intramolecular esterification using a stationary bed flow type reaction apparatus, a lactone can be easily obtained as a product from the trapped solution by merely distilling off the solvent therefrom.

The present catalyst can be reused in the reaction for the production of the above lactone.

According to the present invention, there is provided hydrous zirconium oxide modified with an organosilicon compound, wherein 50-90 % of the hydroxyl groups on the surface of the hydrous zirconium oxide are substituted by the organosilicon compound. For formation of aldehydes or ketones by oxidation of alcohols or for production of lactones, the hydrous zirconium oxide can be used as a catalyst for efficient synthesis of the aldehydes or ketones, or lactones.

EXAMPLES

The present invention is described in more detail with reference to the following examples, which however are not intended to limit the scope of the invention.

Example 1 Production of the hydrous zirconium oxide of the invention (1)

Hydrous zirconium oxide (5.0 g) (purchased from Yuki Gosei Yakuhin Kogyo Co., Ltd. or Dai-ichi Ki-Genso Co., Ltd.) having been calcined at 300 °C for 5 hours was immersed overnight in trimethylsilyl chloride (5.0 g) dissolved in hexane (10 ml), and the solids in the mixture were separated by filtration, washed repeatedly with each of hexane and acetone, and air-dried.

Then, the product was further dried under vacuum to give trimethylsilyl-modified hydrous zirconium oxide, i.e. the hydrous zirconium oxide of the invention.

XPS analysis (X-ray photoelectric spectroscopy) (maximal vacuum degree of $10^{-7}$ millibar, ESCALAB5 manufactured by VG Co., Ltd.) indicated 0.047 of Si/Zr on the surface of the resulting catalyst.

Subsequently, the surface hydroxyl groups on hydrous zirconium oxide of the invention were quantified in the following manner.

The above hydrous zirconium oxide of the invention was introduced to 2.5 (W/V) % lithium hydroxide aluminum in THF, and the hydrogen gas generated was determined with a THF manometer.

The reaction proceeds as in reaction (3):

$$LiAlH_4 + nR\text{-}OH \rightarrow LiAl(OR)_nH_{4-n} + nH2 \tag{3}$$

The experiment apparatus was thermostated at 30 °C, and a predetermined amount of the air in the THF manometer was drawn for determination of hydrogen gas content.

Using the amount of hydrogen generated, the amount of OH groups in mole is calculated in equation (4):

$$n = PV/RT = (p/760) \times V \times (1/0.0821) \times (1/T) \times 10^{-3}$$
$$= pV \times 5.289 \times 10^{-6} \qquad (4)$$

p:      atmospheric pressure (mmHg) V: generated hydrogen (ml)
T:      absolute temperature (K) (30 °C = 303 K).

The measurement result of surface hydroxyl groups of the hydrous zirconium oxide of the invention is set forth in Table 1. The hydrous zirconium oxide not modified with trimethylsilyl group was used as the control.

Table 1

| Amount of hydroxyl groups on the surface of the hydrous zirconium oxide | |
| --- | --- |
| sample | number of OH groups per g (mole number) |
| hydrous zirconium oxide | $1.53 \times 10^{-3}$ |
| trimethylsilyl-modified hydrous zirconium oxide | $2.78 \times 10^{-4}$ |

From this result, it has become apparent that the hydroxyl groups on the trimethylsilyl-modified hydrous zirconium oxide are reduced to less than 1/5 relative to those on the hydrous zirconium oxide. From the surface analysis, it can be concluded that trimethylsilyl groups are covalently bound onto the surface of the hydrous zirconium oxide. In the trimethylsilyl- modified hydrous zirconium oxide, about 80 % of the hydroxyl groups on the surface were modified with trimethylsilyl groups.

The oxidation of alcohol according to the present invention is illustrated in the following examples.

Example 2 Production of the hydrous zirconium oxide of the invention (2)

3-aminopropyltriethoxysilane (10 g) was dissolved in dry toluene (60 ml). 5.0 g of the hydrous zirconium oxide calcined at 300 °C for 5 hours was added thereto and refluxed for 8 hours.

The solids in the solution were then separated by filtration, washed with toluene and air-dried.

This product was further dried under vacuum to give the hydrous zirconium oxide of the invention, i.e. hydrous zirconium oxide modified with 3-aminopropyltriethoxysilane.

Example 3 Alcohol oxidation using the hydrous zirconium oxide of the invention as a catalyst

A. In the presence of the trimethylsilyl chloride-modified hydrous zirconium oxide as a catalyst obtained in Example 1, octyl aldehyde was produced from octyl alcohol in the following manner.

0.1 g of the catalyst obtained in Example 1, 13.0 mg (0.1 mmol) octyl alcohol, and 10 mg of dodecane as an internal standard were added to 5 ml of 5 mmol (0.53 g) benzaldehyde in xylene, and the mixture was allowed to react for 6 hours under reflux.

The product was analyzed by gas chromatography and gas chromatography-mass spectrometric analysis (GC-MS).

The product was found to be an existing substance (commercially available as a standard sample) as a result of comparison between the product and a commercial standard sample in gas chromatography and GC-MS. In the following examples, too, a product was identified by comparison with a standard sample.

As a result, the product was identified as octyl aldehyde.

Gas chromatography analysis indicated 67 % yield.

B. 0.1 g of the catalyst obtained in Example 1, 13.0 mg (0.1 mmol) octyl alcohol, and 10 mg dodecane as an internal standard were introduced into 5 ml of 5 mmol (0.43 g) pivalaldehyde in xylene, and the mixture was allowed to react for 4.5 hours under reflux.

The product was analyzed by gas chromatography and GC-MS.

As a result, the product was identified as octyl aldehyde.

Gas chromatography analysis indicated 43 % yield.

C. 0.2 g of the catalyst obtained in Example 1, 15.8 mg (0.1 mmol) decyl alcohol, and 10 mg dodecane as an internal standard were introduced into 5 ml of 5 mmol (0.53 g) benzaldehyde in xylene, and the mixture was allowed to react for 7 hours under reflux.

The product was analyzed by gas chromatography and GC-MS.

As a result, the product was identified as decyl aldehyde.

Gas chromatography analysis indicated 64 % yield.

D. Oxidation was carried out in the same manner and conditions as in described above in C except that 5 mmol (0.6 g) m-tolualdehyde was used in place of 5 mmol (0.53 g) benzaldehyde.

The product was analyzed by gas chromatography and GC-MS.

As a result, the product was identified as decyl aldehyde.

Gas chromatography analysis indicated 70 % yield.

E. Reaction was carried out in the same manner and conditions as described above in C except that 14.2 mg (0.1 mmol) 3-cyclohexyl-1-propanol was used in place of 15.8 mg (0.1 mmol) decyl alcohol.

The product was analyzed by gas chromatography and GC-MS.

As a result, the product was identified as 3-cyclohexylpropyl aldehyde.

Gas chromatography analysis indicated 57 % yield.

F. 0.2 g of the catalyst prepared in Example 1, 14.2 mg (0.1 mmol) 3-cyclohexyl-1-propanol and 10 mg dodecane as an internal standard were added to 5 ml of 5 mmol (0.43 g) pivalaldehyde in xylene, and the mixture was allowed to react for 4.5 hours under reflux.

The product was analyzed by gas chromatography and GC-MS.

As a result, the product was identified as 3-cyclohexylpropyl aldehyde.

Gas chromatography analysis indicated 45 % yield.

G. Reaction was carried out in the same manner and conditions as described above in C except that 17.6 mg (0.1 mmol) dodecyl alcohol was used in place of 15.8 mg (0.1 mmol) decyl alcohol.

The product was analyzed by gas chromatography and GC-MS.

As a result, the product was identified as dodecyl aldehyde.

Gas chromatography analysis indicated 42 % yield.

H. 10.0 mg (0.1 mmol) cyclohexanol and 10 mg dodecane as an internal standard were added to a mixture of 0.5 ml acetone and 4.5 ml benzene, and the mixture was refluxed for 10 hours in the presence of 0.1 mg of the above catalyst.

The product was analyzed by gas chromatography and GC-MS.

As a result, the product was identified as cyclohexanone.

Gas chromatography analysis indicated 75 % yield.

a. Reaction was carried out in the same manner and conditions as described in A except that 0.1 g of hydrous zirconium oxide not modified was used in place of the catalyst obtained in Example 1.

The product was analyzed by gas chromatography and GC-MS.

As a result, the yield of octyl aldehyde was found to be 36 %, which is significantly lower than the 67 % yield of octyl aldehyde described above in A.

b. Reaction was carried out in the same manner and conditions as described above in C except that 0.2 g of hydrous zirconium oxide not modified was used in place of the catalyst described in C.

The product was analyzed by gas chromatography and GC-MS.

As a result, the yield of decyl aldehyde was found to be 19 %, which is significantly lower than the 64 % yield of decyl aldehyde described above in C.

c. Reaction was carried out in the same manner and conditions as described in H except that 0.4 g of hydrous zirconium oxide not modified was used in place of the catalyst of the invention and that the reflux time was 1 hour.

The product was analyzed by gas chromatography and GC-MS.

As a result, the yield of cyclohexanone was found to be 52 %, which is significantly lower than the 75 % yield of cyclohexanone described above in H.

Example 4 Production of lactone using the hydrous zirconium oxide of the invention as a catalyst

A. 0.5 g of the catalyst prepared in Example 1 was charged such that a catalyst layer was immobilized uniformly inside a glass reaction tube of 8 mm diameter.

The reaction tube was placed in an electric oven in which the temperature was kept at 250 °C.

Separately, a toluene solution containing 1.9 g (12 mmol) ethyl 6-hydroxyhexanoate and 0.1 g (0.6 mmol) dodecane was prepared as a starting material solution to be supplied for synthesis.

The starting material solution was evaporated and past together with a carrier gas through the catalyst layer in the above reaction tube.

The carrier gas used was a nitrogen gas at a flow rate of 1 ml/sec and added and supplied at a rate of 5 ml/hour by a microfeeder.

The product gas, after past along with the carrier gas, was lead outside the oven and condensed by cooling to be liquefied for recovery.

The recovered product was analyzed by gas chromatography and gas chromatography-mass spectrometric analysis (GC-MS).

As a result, the product was identified as $\varepsilon$-caprolactone (6-hexanolide).

Gas chromatography analysis indicated 92 % yield.

B. Reaction was carried out in the same manner and conditions as described above in A except that the amount of the catalyst used was 1.0 g, and that 0.21 g (1.2 mmol) ethyl 7-hydroxyheptanoate was used in place of ethyl 6-hydroxyhexanoate.

The product was analyzed by gas chromatography, GC-MS, gas chromatography-infrared spectroscopy (GC-IR) and $^1$H-NMR.

As a result, the product was identified as 7-heptanolide.

Gas chromatography analysis indicated 80 % yield.

C. Reaction was carried out in the same manner and conditions as described above in B except that 0.26 g (1.2 mmol) ethyl 6-hydroxydecanoate was used in place of ethyl 7-hydroxyheptanoate.

The product was analyzed by gas chromatography, GC-MS, gas chromatography-infrared spectroscopy (GC-IR) and $^1$H-NMR.

As a result, the product was identified as 6-decanolide.

Gas chromatography analysis indicated 50 % yield.

## Claims

1. Hydrous zirconium oxide modified with an organosilicon compound, wherein 50-90 % of the surface hydroxyl groups on the hydrous zirconium oxide are substituted by the organosilicon compound.

2. A catalyst for the oxidation of alcohols, comprising as a constituent the hydrous zirconium oxide modified with an organosilicon compound as defined in claim 1.

3. A catalyst for the production of lactones, comprising as a constituent the hydrous zirconium oxide modified with an organosilicon compound as defined in claim 1.

## Patentansprüche

1. Mit einer Organosiliziumverbindung modifiziertes, wasserhaltiges Zirkoniumoxid, worin 50-90% der Oberflächen-Hydroxylgruppen auf dem wasserhaltigen Zirkoniumoxid durch die Organosiliziumverbindung substituiert sind.

2. Katalysator für die Oxidation Von Alkoholen, umfassend als einen Bestandteil das mit einer Organosiliziumverbindung modifizierte, wasserhaltige Zirkoniumoxid gemäß Anspruch 1.

3. Katalysator für die Herstellung von Lactonen, umfassend als einen Bestandteil das mit einer Organosiliziumverbindung modifizierte, wasserhaltige Zirkoniumoxid gemäß Anspruch 1.

## Revendications

1. Oxyde de zirconium hydraté modifié par un composé organique du silicium, dans lequel 50 à 90 % des groupes hydroxyle de surface sur l'oxyde de zirconium hydraté sont substitués par le composé organique du silicium.

2. Catalyseur pour l'oxydation d'alcools, comprenant, en tant que constituant, l'oxyde de zirconium hydraté modifié par un composé organique du silicium et tel que défini dans la revendication 1.

3. Catalyseur pour la production de lactones, comprenant, en tant que constituant, l'oxyde de zirconium hydraté modifié par un composé organique du silicium et tel que défini dans la revendication 1.